(19) Europäisches Patentamt
European Patent Office
Office européen des brevets

(11) Publication number: **0 089 743**
**B1**

(12) **EUROPEAN PATENT SPECIFICATION**

(45) Date of publication of patent specification: **10.12.86**

(51) Int. Cl.[4]: **C 07 D 245/06,** A 61 K 31/395

(21) Application number: **83300788.3**

(22) Date of filing: **16.02.83**

(54) **Novel 2,5-benzodiazocines and salts thereof having pharmaceutical activity.**

(30) Priority: **24.03.82 JP 45716/82**

(43) Date of publication of application:
**28.09.83 Bulletin 83/39**

(45) Publication of the grant of the patent:
**10.12.86 Bulletin 86/50**

(84) Designated Contracting States:
**DE FR GB IT**

(56) References cited:
**DE-A-1 645 963**
**FR-A-1 487 344**
**JOURNAL OF MEDICINAL CHEMISTRY, vol. 21, no. 8, August 1978, pages 758-763, Columbus, Ohio, US**
**H. H. ONG et al,: "Synthesis and analgetic activity of some 5-aryl-2-azabicyclo(3.2.1)octanes"**
**A. BURGER, Medicinal Chemistry, Fourth Edition, Part III, John Wiley, New York, p. 740(1981)**
**A. BURGER, Medicinal Chemistry, Third Edition, Part I, Wiley-Interscience, New York, page 77 (1970)**
**E. J. ARIENS, Drug Design in Medicinal Chemistry, A Series of Monographs, vol. 1,**

(73) Proprietor: **SANWA KAGAKU KENKYUSHO CO., LTD.**
**No. 35, Higashi-sotobori-cho**
**Higashi-ku Nagoya-shi Aichi-ken (JP)**

(72) Inventor: **Ban, Masatoshi**
**5-40, Kanosakaecho-dori**
**Gifu-shi Gifu-ken (JP)**
Inventor: **Miura, Kenji**
**2-59, Sekita-cho**
**Kasugai-shi Aichi-ken (JP)**
Inventor: **Baba, Yutaka**
**56, Aza-tomoura**
**Akechi Bisai-shi Aichi-ken (JP)**
Inventor: **Iwata, Noriyuki**
**10-197-3, Kamijo-cho**
**Kasugai-shi Aichi-ken (JP)**
Inventor: **Fukui, Akira**
**10-197-3, Kamijo-cho**
**Kasugai-shi Aichi-ken (JP)**
Inventor: **Hori, Mikio**
**5-33-1, Hon-cho**
**Gifu-shi Gifu-ken (JP)**
Inventor: **Fujimura, Hajime**
**9-2, Shishigatani -shimomiyanomae-cho**
**Sakyo-ku Kyoto-shi Kyoto-fu (JP)**
Inventor: **Suenaga, Eiichi**
**1-15-20, Nishi**
**Kunitachi-shi Tokyo (JP)**

**EP 0 089 743 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
pages 241-263, particulary page 243, 1971
C. W. THORNBER, Isosterism and Molecular Modification in Drug Design in "Chem. Soc. Review", vol. 8, pages 563-580, 1979

E. J. ARIENS, Drug Design, volume I; Academic Press, New York, pages 101-121. 1971

(74) Representative: **Moore, Anthony John et al**
**Gee & Co. Chancery House Chancery Lane**
**London WC2A 1QU (GB)**

**Description**

This invention relates to a class of novel 2,5-benzodiazocines and salts thereof, and to pharmaceutical agents comprising as an active component at least one such benzodiazocine or salt.

According to the present invention there is provided a 2,5-benzodiazocine represented by the following general formula (I):

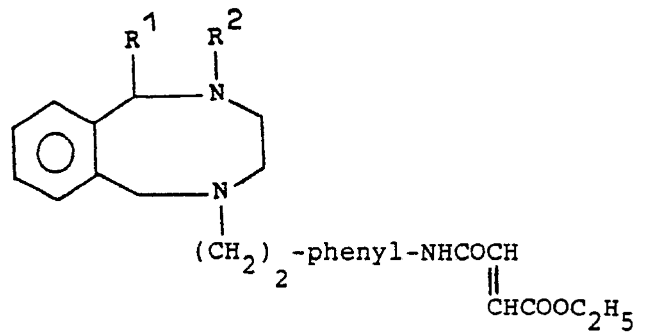

(I)

wherein $R^1$ is phenyl or p-methoxyphenyl and $R^2$ is hydrogen or methyl or a salt thereof.

The compounds represented by the general formula (I) and salts thereof display various actions on the nervous system, particularly analgesic and antipyretic actions, and usually antitussive and sleep-depth increasing actions, and thus are of use as analgesics and antipyretics, and usually also as antitussives and sedatives.

The compounds of the formula (I) are novel compounds which have not been discolsed in any prior art literature and according to the invention, can be prepared from, for example, a 1-substituted or unsubstituted phenyl-1,2,3,4,5,6-hexahydro-2,5-benzodiazocine represented by the following general formula (II):

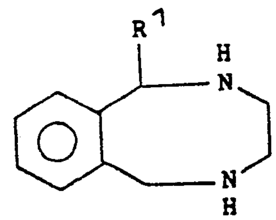

(II)

wherein $R^1$ has the previously-defined meaning, substituting an organic radical for the 5-hydrogen atom in a manner known per se such as by the acid chloride, carboxylic acid or alkylhalogenide method, and if necessary methylating at the 2-position in a manner known per se. A suitable protective radical may selectively be used for the methylation at the 2-position.

The following Compound (I-a) is exemplary of those represented by the general formula (I):

[5(E)]-ethyl-4-[[4-[2-(1,2,3,4,5,6-hexahydro-1-phenyl-2,5-benzodiazocin)-5-yl]ethyl]phenyl]amino-4-oxo-2-butenoate

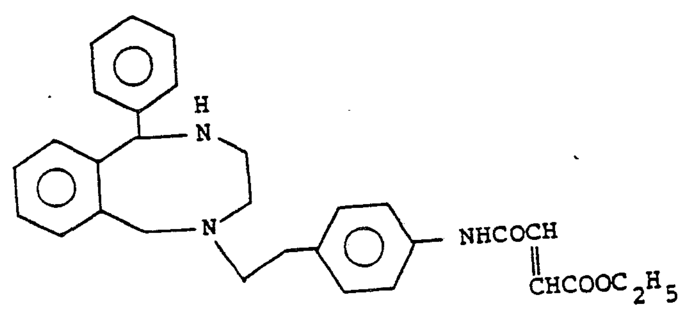

(I-a)

The compounds of the formula (I) and pharmaceutically-accepatble salts thereof can be administered injectionally, orally or by any conventional route. The dosage for the treatment of an adult varies depending on the administrative route and frequency but is about from 5 to 200 mg per day and for instance, in oral dosage, 30 to 40 mg per time is preferable for an adult.

The invention will now be further illustrated in detail, with reference to the following Examples.

Example

[5(E)]-ethyl-4-[[4-[2-(1,2,3,4,5,6-hexahydro-1-phenyl-2,5-benzodiazocine)-5-yl]ethyl]phenyl]amino-4-oxo-2-butenoate: Compound (I-a) (hydrochloride)

A mixture of 8.00 g (33.6 mmol) of 1,2,3,4,5,6-hexahydro-1-phenyl-2,5-benzodiazocine, 8.51 g (37.0 mmol) of p-nitrophenethylbromide, 4.23 g (50.4 mmol) of $NaHCO_3$ and 60 ml of dimethylformamide was stirred at 100°C for 10 hours under an inert gas atmosphere and then subjected to distillation to remove the solvent therefrom. After addition of 150 ml of water, the reaction mixture was extracted with $CHCl_3$, washed with water, dried on $Na_2SO_4$, subjected by distillation to remove the solvent and refined by silica gel column chromotography (ethyl ether/n-hexane = 1/1; triethylamine/ethyl ether = 1/10) to obtain 10.5 g (80.7%) of nitrophenethyl compound. 5.54 g (14.3 mmol) of this compound were dissolved in 25 ml of $CHCl_3$ and 150 ml of methanol were added thereto. After addition of 1 g of 10% Pd-C under an inert gas atmosphere, the mixture was stirred at room temperature for 3 hours under a hydrogen atmosphere and then filtered off the catalyst. The filtrate was concentrated under reduced pressure, treated with hydrogen chloride in methanol to form the hydrochloride and then the hydrochloride recrystallized from methanol/methylethylketone to obtain 6.60 g (99.0%) of aminophenethyl compound as colorless prismatic crystals having melting point of 252—258°C (dec.).

2.93 g (8.21 mmol) of the free base of the compounds were dissolved in 100 ml of tetrahydrofuran and then 30 ml of a tetrahydrofuran solution of 1.47 g (9.03mmol) of ethyl-3-(chloroformyl)acrylate were added thereto dropwise over 30 minutes at 0°C. After having been stirred 1 hour, the reaction mixture was concentrated under reduced pressure and 100 ml of water were added thereto. The solution was made alakaline with ammonia, extracted with $CHCl_3$, washed wth water, dried over $Na_2SO_4$, subjected to distillation to remove the solvent and refined by silica gel column chromotography (triethylamine/ethyl acetate/n-hexane = 0.5/1.5—0.3/2/1) to obtain 2.97 g (74.8% of the objective compound (fee base). The free base was treated by hydrogen chloride in methanol and recrystallized from water/methanol/ethylethylketone to obtain the desired compound (hydrochloride) as colorless prismatic crystals having a melting point of 248—255°C (dec.). This salt could also be converted into the free base in a conventional manner.

Elementary Analysis: $C_{30}H_{33}N_3O_3 \cdot 2HCl$
Cal. C 64.75, H 6.34, N 7.55
Found C 64.52, H 6.49, N 7.26

IR($\nu_{max}^{KBr}$)$cm^{-1}$
2480 ($NH^+$), 1700, 1675 (C = O)

MS: EI/DI (m/z); 483 ($M^+$), 194 (base)
CI/DI (i-Bu) (m/z); 484 (M+1)
High MS (m/z); $C_{30}H_{33}N_3O_3$ ($M^+$)
Cal. 483, 2522
Found 483, 2534

NMR ($CDCl_3$) δ ppm:
8.40 (1H, br, N<u>H</u>—CO, disappear by adding $D_2O$)
7.7—6.6 (15H, m, Ar—H and olefinic proton)
5.53 (1H, s, C1—H)
4.28 (1H, ABq, J=13.OHz, $\Delta\nu$=20Hz, C6—$H_2$)
4.25 (2H, 1, J=7.0Hz, —C<u>H</u>$_2$CH$_3$)
3.2—2.5 (8H, m, —$CH_2$— and $NCH_2CH_2Ar$)
1.93 (1H, br-s, NH, disappear by adding $D_2O$)
1.30 (3H, t, J=7.0Hz, —CH$_2$C<u>H</u>$_3$)

Pharmaceutical Tests
A) Analgeisic Action
i) Haffner's Method

In accordance with the usual procedure, dd-male mice of 14 to 22 g were classified into groups of ten mice each, and the compounds to be tested in various concentrations were given to respective mice. A Koffer clamp was then clamped to the tail root of each mouse after periods of 15, 30, 45 and 60 minutes had elapsed from the time of each administration to measure analgesic action of the compounds which was judged as "negative" when the tested mouse cried or looked back.

Further, the number of deaths in each group of ten mice was counted after two hours from the time of administration of the testing agent into abdominal cavity had elapsed; $ED_{50}$, $LD_{50}$ and 95% confidence limit were then calculated in accordance with the Richfield-Wilcoxson Method to compare the results obtained by the use of the agents.

ii) Acetic Acid Stretching Method

dd-Male mice of 17 to 23 g weight were classified into groups, each of ten mice, a 0.7% aqueous solution of acetic acid was adminstered into the abdominal cavity of each mouse in an amount of 10 ml/kg and then the stretching condition was observed over a period of 5 minutes after 10 to 15 minutes from the time of dosage had elapsed. The test was repeated four times to obtain a total measured value and the inhibition ratio was measured from a number of stretching actions. Observations have been made on a "Yes" or "No" system.

$ED_{50}$, $LD_{50}$ and 95% confidence limit were calculated according to the Richfield-Wilcoxson Method and the results compared for each testing agent and control.

Each testing agent and control were given orally 30 minutes before the administration of the acetic acid in an amount of 100 mg/10 ml/kg.

B) Antitussive Action

Into the abdominal cavity of each of a group of Hartley type guinea pigs having a body weight of from 300 to 450 g, there was administered 0.5 ml/kg of menbutal to induce light anaesthesia. Thereafter, each trachea was exposed by a surgical operation on the throat and a pinhole made through the trachea so as to allow insertion of a hog bristle. Those guinea pigs who coughed each time the bristle was inserted into the pin-hole after lapses of 5 and 20 minutes from the time of the surgical operation were employed for the test. Measurements of the antitussive action were carried out after 30, 45, 60, 90 and 120 minutes had elapsed from the time that the agents to be tested had been administered into abdominal cavity in an amount of 2.5 ml/kg (50 mg/kg). The inhibition ratio was determined based on total measured value over six tests and the value measured before administration.

The inhibition ratio was classified into the following five classes:—

| | | |
|---|---|---|
| ( − ) | Inhibition Ratio, | 0% |
| ( ± ) | Inhibition Ratio, | 0—10% |
| ( + ) | Inhibition Ratio, | 10—20% |
| (++) | Inhibition Ratio, | 20—35% |
| (+++) | Inhibition Ratio, | more than 35% |

C) Antipyretic Action

Wister type male rats of 100 to 140 g body weight were classified into groups, each of four to five rats. The rats were abstrained from food for one day and then each rat was put into a separate cage. The body temperature was measured through the rectum under a constant room temperature. This body temperature measurement was carried out three times before administration of the agents as well as after 30, 60, 90, 120 and 150 minutes from the time of dosage had elapsed.

Observations were made based on following five criteria:—

(−) clearly showing no decrease in body temperature;
(±) decrease in body temperature but no noticeable difference recognizable;
(+) decrease in body temperature of 1.5°C and at least one result showing a notifiable difference of $P<5\%$ obtained, or decrease in body temperature of 1°C only but the value of $P<1\%$;
(++) decrease in body temperature of 1 to 2°C and at least one result showing a notifiable difference of $P<0.1\%$ obtained.

D) Effect on Sleep

dd-Type male mice of 16 to 24 g body weight were classified into groups, each of ten mice. A testing agent was administered through the abdominal cavity of each mouse, and after a lapse of 30 minutes from the time of administration, 40 mg/kg of pentobarbital was also administered through the abdominal cavity to measure the length of the sleeping period, which is defined as the period in which a facial reflex disappears. Observation were recorded on the basis of the length of the period of sleep in comparison with the control, as follows:—

(−−−) sleeping period shortened by 60 minutes more;
(−−) sleeping period shortened by 30 to 60 minutes;
(−) sleeping period shortened by 10 to 30 minutes;
(±) sleeping period shortened or extended by ± 10 minutes;
(+) sleeping period extended by 10 to 30 minutes;
(++) sleeping period extended by 30 to 60 minutes;
(+++) sleeping period extended by 60 minutes or more.

E) Acute Toxicity

Five-week old dd-type male mice of 21 to 26 g body weight and five-week old Wister type male rats of 110 to 150 g body weight wer classified into groups, each of ten mice or rats. After administration of testing agents to the general condition of the mice and rats, the number of deaths each day and body weight changes were observed over 7 days. An $LD_{50}$ value was calculated based on the total number of deaths during the seven-day testing period, in accordance with the Richfield-Wilcoxson Method. The surviving animals were dissected to enable visual observation of their internal organs.

The following Tables show the results obtained from the tests described above.

The agents according to the invention employed in the tests were hydrochloride of the Compounds identified in the Tables.

| Compounds or agent (Example No.) | Analgesic Action | | | | Antitussive Action | Antipyretic Action | |
|---|---|---|---|---|---|---|---|
| | Acetic Acid Stretching Method | | Haffner's Method | | In abdominal cavity | In abdominal cavity | |
| | | $ED_{50}$ | | $ED_{50}$ | 50 mg/Kg | 25 mg/Kg | 50 mg/Kg |
| I-a | 32.6~ 114.4 | 61.1 | 52.7~ 122.4 | 80.3 | – | | ++ |
| Nefopam (control) | | | | | ++ (25 mg/Kg) | ++ | |

| Compound or agent | Sleep Incrementing Action | | Acute Toxicity (mg/Kg) | | | | |
|---|---|---|---|---|---|---|---|
| | Oral dosage | In abdominal cavity | Rat | | Mouse | | |
| (Example No.) | 100 mg/Kg | 50 mg/Kg | Oral dosage | Intravenous injectional dosage | Oral dosage | Intravenous injectional dosage | In abdominal cavity |
| I-e | ++ | — | >5000 | — | 938 (751~ 1172) | — | >200 |
| Nefopam (control) | — (50 mg/Kg) | — (25 mg/Kg | 595 (492~ 720) | 18.0 (16.5~ 19.6) | 356 (306~ 414) | 36.8 (33.6~ 40.3) | 72.4 (69.5~ 75.4) |

## Claims

1. A 2,5-benzodiamine represented by the following general formula (I):

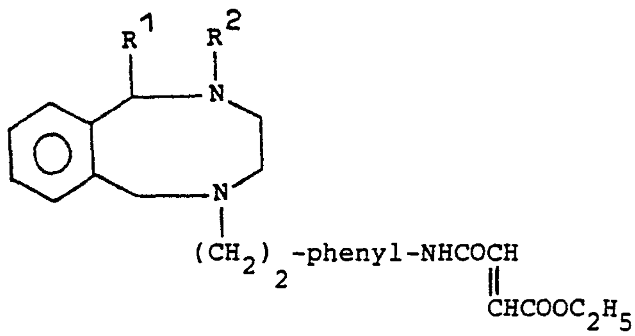

(I)

wherein $R^1$ is phenyl or p-methoxyphenyl and $R^2$ is hydrogen or methyl or a salt thereof.

2. A compound as claimed in Claim 1 and having the following structural formula (I-a) or a salt thereof:

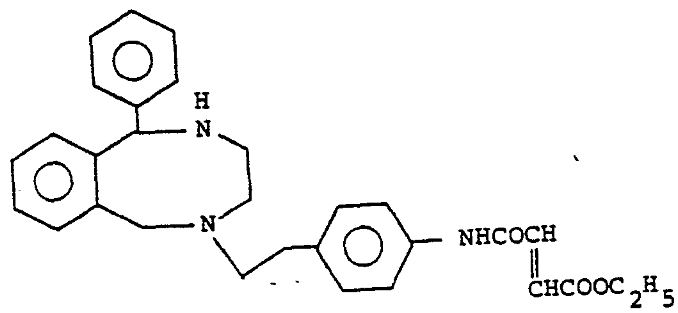

(I-a)

3. A process for preparing a 2,5-benzodiazocine as claimed in Claim 1, comprising introducing onto the 5-nitrogen atom of a compound represented by the following general formula (II):

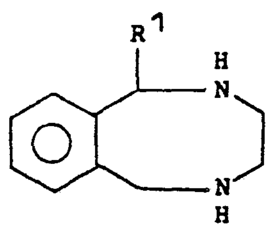

(II)

wherein $R^1$ is phenyl or p-methoxyphenyl, a group of the formula

$$—(CH_2)_2—phenyl—NHCOCH \\ \| \\ CHCOOC_2H_5$$

and optionally methylating the 2-nitrogen atom.

4. A pharmaceutical composition which comprises, as an active ingredient, at least one 2,5-benzodiazocine as claimed in Claim 1 or 2 or a pharmaceutically-acceptable salt thereof and a pharmaceutically-acceptable carrier.

5. A pharmaceutical composition in unit dosage form for administration orally or by injection and containing a 2,5-benzodiazocione as claimed in Claim 1 or 2 or a pharmaceutically-acceptable salt thereof.

**Patentansprüche**

1. 2,5-Benzodiazocin, gekennzeichnet durch folgende allgemeine Formel (I):

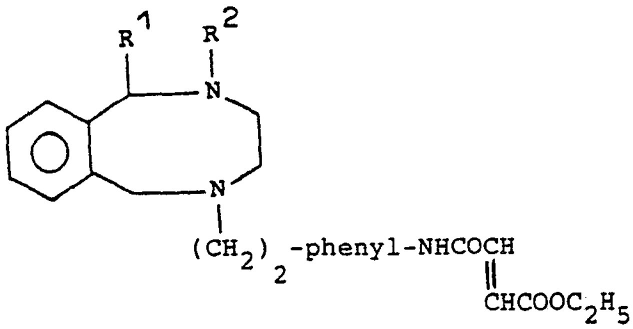

(I)

worin $R^1$ ein Phenyl oder p-Methoxyphenyl, und $R^2$ Wasserstoff, ein Methyl oder ein Salz davon darstellt.

2. Verbindung nach Anspruch 2, gekennzeichnet durch folgende Strukturformel (I-a) oder einem Salz davon:

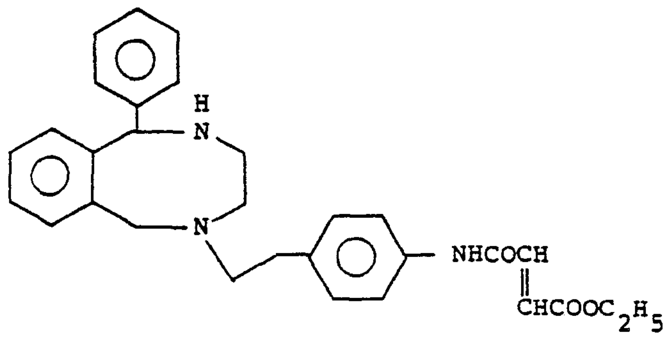

(I-a)

3. Verfahren zur Herstellung eines 2,5-Benzodiazocins gamäß Anspruch 1, dadurch gekennzeichnet, daß auf das 5-Stickstoffatom der durch die nachstehende allgemeine Strukturformel (II) verkörperte Verbindung:

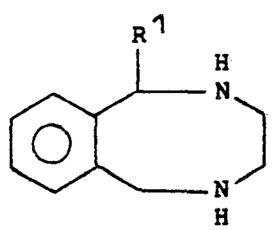

(II)

worin $R^1$ ein Phenyl oder p-methoxyphenyl darstellt eine Gruppe der Formel:

$-(CH_2)_2$-phenyl-NHCOCH
||
$CHCOOC_1H_5$

eingeführt wird, und das 2-Stickstoffatom wahlweise methylisiert wird.

4. Pharmazeutische Verbindung, dadurch gekennzeichnet, daß diese als aktiven Bestandteil mindestens ein 2,5-Benzodiazocin gemaß Anspruch 1 oder 2 oder ein pharmazeutisch akzeptables Salz davon sowie einen pharmazeutisch akzeptablen Trägerstoff beinhaltet.

5. Pharmazeutische Verbindung in einheitlicher Dosis zur oralen Verabreichung oder zur Injektion, dadurch gekennzeicnet, daß diese ein 2,5-Benzodiazocin gemäß Anspruch 1 oder 2 ein pharmazeutisch akzeptables Salz davon enthält.

**Revendications**

1. 2,5-benzodiazocine représentée par la formule générale suivante (II):

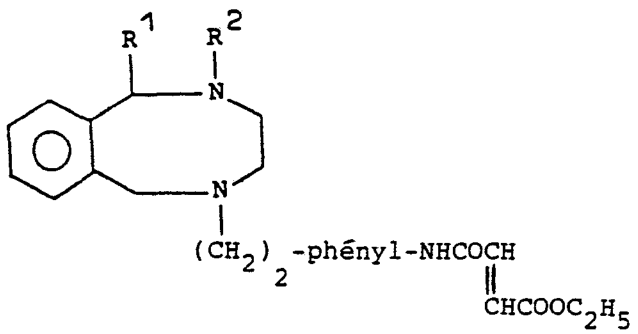

(I)

dans laquelle $R^1$ est du phényle ou du p-méthoxyphényle et $R^2$ est de l'hydrogène ou du méthyle ou un sel de celui-ci.

2. Composé selon la revendication 1 ayant la formule de structure suivante (I-a) ou un sel de ce composé:

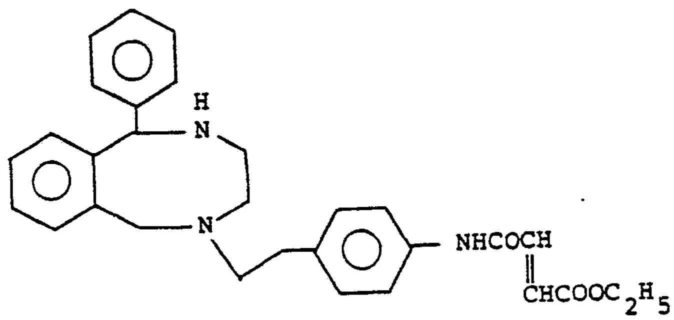

(I-a)

3. Procédé pour préparer une 2,5-benzodiazocine selon la revendication 1, consistant à introduire sur l'atome d'azote en position 5 d'un composé représenté par la formule générale suivante (II):

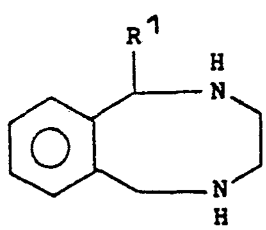

(II)

dans laquelle $R^1$ est du phényle ou du p-méthoxyphényle, un groupe de la formule:

$$-(CH_2)_2\text{-phenyl-NHCOCH}=\text{CHCOOC}_1H_5$$

et, au choix, à méthyler l'atome d'azote en position 2.

4. Composition pharmaceutique, qui contient, comme ingrédient actif, au moins une 2,5-benzodiazocine selon la revendication 1 ou 2, ou un sel de celle-ci pharmacetiquement acceptable.

5. Composition pharmaceutique sous forme de dosage unitaire pour adminstration orale ou par injection et contenant une 2,5-benzodiazocine selon la revendication 1 ou la revendication 2, ou un sel de celle-ci pharmacetiquement acceptable.